# EUROPEAN PATENT SPECIFICATION

(11) **EP 1 352 246 B1**
(45) Date of publication and mention of the grant of the patent: **03.12.2008**
(21) Application number: 01273004.0
(22) Date of filing: 27.12.2001
(51) Int. Cl.: G01N 33/68, G01N 33/536, G01N 33/564

(54) **USE OF A PURE ALLERGEN COMPONENT**
VERWENDUNG EINER REINEN ALLERGENKOMPONENTE
UTILISATION D'UN COMPOSANT ALLERGENE PUR

(30) Priority: 29.12.2000 SE 0004891
(43) Date of publication of application: 15.10.2003
(73) Proprietor: Phadia AB, 751 37 Uppsala (SE)
(72) Inventor: STUMVOLL, Sabine, A-3352 St. Peter (AT); LIDHOLM, Jonas, S-741 42 Knivsta (SE); WESTRITSCHNIG, Kerstin, A-1180 Vienna (AT); SPITZAUER, S. Inst. of Med. and Chem. Lab. Diagn., A-1090 Vienna (AT); KRAFT, Dietrich, A-1170 Vienna (AT); GERACI, D. Istituto di Biologia dello Sviluppo, I-90146 Palermo (IT); VALENTA, Rudolf, A-2604 Theresienfeld (AT); COLOMBO, P. Istituto di Biologia dello Sviluppo, I-90146 Palermo (IT); DURO, G. Istituto di Biologia dello Sviluppo, I-90146 Palermo (IT)
(74) Representative: Lindgren, Anders
(86) International application number: PCT/SE2001/002907
(87) International publication number: WO 2002/054082

(56) References cited:
- EP-A2- 0 707 065
- WO-A1-99/51988
- US-A- 5 770 698
- US-A- 5 786 161
- GIOVANNI DURO ET AL.: 'cDNA cloning, sequence analysis and allergological characterization of Par j 2.0101, a new major allergen of the parietaria judaica pollen' FEBS LETTERS vol. 399, 1996, pages 295 - 298, XP002909979
- DATABASE BIOSIS [Online] D'ABUSCO ET AL.: 'Characterization of a dominant antigenic determinant of Par o I encoded by recombinant DNA', XP002909980 Database accession no. PREV199698721445 & CLINICAL AND EXPERIMENTAL ALLERGY vol. 26, no. 2, 1996, pages 223 - 231
- STUMVOLL SABINE ET AL.: 'Recombinant Par J 2, a marker allergen for mediterranean sensitization' JOURNAL OF ALLERGY AND CLINICAL IMMUNOLOGY, [Online] vol. 107, no. 2, February 2001, XP002959037 Retrieved from the Internet: <URL:http://www2.us.elsevierhealt.com/scrip ts/om.dll/serve?id=aai011072b1&action=.sear ...> [retrieved on 2003-03-13]

## Description

### Field of the invention

The present invention relates to the use of an isolated allergen component in a serological assay for improved precision in identification of sensitizing allergen source in the diagnosis of allergy. This enables not only adequate measures for avoidance of the causative agent but also appropriate selection of treatment of the allergic disease.

### Background of the invention

About 20 % of the western world population suffer from Type I allergy, a hypersensitivity disease involving the formation of IgE antibodies against otherwise harmless molecular structures present in the environment, i.e. allergens. The symptoms of Type I allergy (allergic rhinitis, conjunctivitis and allergic asthma) are mainly caused by the crosslinking of effector-cell bound specific IgE antibodies by allergen molecules, which triggers the release of biological mediators such as histamine and leukotriens that give rise to the tissue reaction.

Diagnosis of allergy includes measures to identify the source of sensitizing substances. For this purpose, common practise is to use extracts of allergen sources either for in vivo challenge, typically in the skin and scoring for tissue reaction, or for serological testing by measuring specific IgE antibodies. In the latter modality, serum from a blood sample is used for quantitative and objective testing against any number of suspected allergens without subjecting the patient to offending allergens. The identifying precision of diagnostic methods utilizing crude allergen extracts is, however, hampered by the extensive patterns of serological relationships that exist between molecular structures present in closely and distantly related species producing allergens.

Thus, sensitization to a particular allergen source can lead to seropositivity to a range of other allergenic materials which the patient may never have been in contact with. Such serological cross-reactivity may or may not be clinically relevant (i.e. capable of eliciting allergic symptom) and can act to confound the identification of offending allergen which forms the basis for meaningful advice on allergen avoidance and, in particular, for the choice of allergen extract for immunotherapy.

Weed pollens constitute important allergen sources worldwide, including prominent examples such as ragweed (*Ambrosia* spp.), mugwort (*Artemisia* vulgaris) and wall pellitory (*Parietaria* spp). Some but not all of the allergens present in pollen of any particular weed species are represented by structurally similar homologues in other species and therefore show some degree of serological cross-reactivity. Thus, depending on the composition of antibody specificities generated by the immunological response in an allergic individual, sensitization to one weed species may lead to serological test positivity also to other species. Serological testing will in such cases of apparent multisensitization generate results that are ambiguous in terms of identification of actual sensitizer.

Using total allergen extracts it is therefore often difficult or impossible to unambiguously determine the allergen source responsible for sensitization and to ensure an optimal choice of selective immunotherapy. Selection of an appropriate allergen source for immunotherapy, which accurately reflects the primary or actual sensitizer, is highly desirable with respect to treatment efficacy, risk of acute side effects and induced additional sensitization, as well as to cost saving and inconvenience to the patient undergoing treatment.

EP 0707 065 describes recombinant *Parietaria* proteins and derived peptides identified as being allergenic. In EP 0707 065, these peptides are contemplated for use in both therapy and diagnosis of *Parietaria* pollen-induced allergy. However, the utility of these proteins/peptides as reagents to distinguish between genuine *Parietaria* pollen sensitization and cross-reaction-mediated seropositivity to *Parietaria* pollen extract is not demonstraded or discussed.

### Summary of the invention

According to the present invention stringent differentiation between different weed pollen allergies is possible for the first time, significantly improving the criteria for accurate choice of selective immunotherapy.

The present invention provides a new use of an isolated allergen component of limited cross-reactivity, to serologically determine sensitizing allergen source, among a variety of possible allergen sources, with higher level of accuracy than is possible using crude allergen extracts. This enables enhanced precision in the choice of allergen extract for selective treatment of a disorder involving an allergen, such as Type I allergy. The treatment may comprise immunotherapy or pharmacotherapy.

Thus, the invention provides use of a pure allergen component selected from the group consisting of Par j 1 and Par j 2 cross-reactivity to serologically identify the actual sensitizing allergen source among a variety of possible allergen sources containing cross-reactive proteins or epitopes. The use of the invention is, for example, valuable for selection of treatment of a disorder involving an allergen selected from the group consisting of Par j 1 and Par j 2.

The allergen component is derived from pollen of a plant species, preferably a weed species, such as mugwort, ragweed or wall pellitory.
A preferred species is *Parietaria judaica,* especially wherein the allergen component is Par j 1 or Par j 2 (1, 2). The method used to generate these components is not crucial for the invention and may be synthetic, recombinant or native.

### Detailed description of the invention

The present invention will now be described in association with an exemplyfing allergen component, i.e. Par j 2.

The present inventors investigated whether recombinant Par j 2, a major *Parietaria judaica* pollen allergen, can be used as a diagnostic tool to identify allergic patients with primary sensitization to *Parietaria,* a mediterranean weed. The present inventors analysed the serum IgE reactivity of patients reactive to total parietaria extracts from Austria (n = 42), Scandinavia (n = 8), USA (n = 18) and Italy (n = 37) to ragweed, mugwort, *Parietaria* pollen extracts and rPar j 2 by immunoblot as well as by CAP RAST measurements. It was found that almost all patients from Scandinavia, USA and Austria contained IgE antibodies to ragweed, mugwort and *Parietaria* pollen extracts. No serum from Scandinavia and USA and only four Austrian sera, but 81 % of the mediterranean patients reacted with rPar j 2. rPar j 2 can therefore be used as a marker allergen to identify allergic patients with a mediterranean sensitization profile who were primarily sensitized to *Parietaria* pollen. rPar j 2 may thus be a useful diagnostic tool for the selection of suitable weed pollen allergen extracts for specific immunotherapy of weed pollen allergic patients

### MATERIALS AND METHODS

### Patients sera

Weed pollen allergic patients from Austria (n = 42), Scandinavia (n = 8), USA (n = 18) and Mediterranian (n = 37) were characterized by skin reactivity and immunoblot. The presence of serum IgE Abs specific for ragweed, mugwort, *Parietaria* pollen extracts and rPar j 2 was confirmed by RAST (radioallergosorbent test) analysis (Pharmacia, Uppsala, Sweden).

### Allergen extracts, Western blotting

Pollen from mugwort (*Artemisia vulgaris*) and *Parietaria* (*Parietaria judaica*) were obtained from Allergon AB (Välinge, Sweden) and stored at 4° C until use.
Two grams of pollen was extracted in 50 ml of distilled water for 2 hours at room temperature. The protein extracts were then centrifuged at 30,000 g for 30 minutes at 4° C. Supernatants were frozen, lyophilized, and checked for protein quantity and quality of proteins by SDS-PAGE and Coomassie blue staining (Bradford et al. 1976). Comparable amounts of each extract (220 µg/cm) were separated by 12 % SDS-PAGE (Fling et al. 1986) and blotted onto nitrocellulose (Schleicher & Schuell, Dassel, Germany) (Towbin et al. 1979).

### rPar j 2 production

The Par j 2 protein was expressed in *E. coli* strain BL21 from a cDNA described by Duro *et al.* (2), cloned in the pET-23a expression vector. Culture medium was inoculated 1 to 500 with an overnight culture and first grown at 30°C to mid-log phase. The incubation temperature was then raised to 42°C for 45 min, followed by 4 hrs at 30°C before harvest. Cells were collected by centrifugation at 10 000 g for 10 min at +4°C and resuspended in 5 ml of buffer A (20 mM Tris-HCl pH 8.0, 0.5 M NaCl, 5 mM imidazole) per gram (fresh weight) of cells. The resuspended cells were ruptured by sonication while kept on ice, followed by centrifugation to remove solid material. The supernatant was loaded onto a Ni²⁺-charged 5 ml HiTrap Chelating column (Amersham Pharmacia Biotech, Uppsala, Sweden) for IMAC. The column was washed with 10 volumes of buffer A containing 20 mM imidazole and elution was performed with a 20-500 mM gradient of imidazole in buffer A. Fractions containing pure rPar j 2 protein were pooled and subjected to buffer exchange to 0.15 M NaCl using a Sephadex G-25 column (Amersham Pharmacia Biotech, Uppsala, Sweden). The concentration of rPar j 2 was determined from absorbance at 280 nm, using a calculated absorption coefficient of 0.24 per mg/ml. The protein was divided into aliquots and stored at -20°C until use.

### Analysis of IgE-binding by rPar j 2 using Pharmacia CAP System

*In vitro* IgE antibody binding by rPar j 2 was examined in Pharmacia CAP System, a clinically used allergy diagnostic immunoassay system. Experimental ImmunoCAP tests were prepared by covalent immobilisation of the purified allergen onto activated cellulose at a concentration chosen so as to achieve an adequate linear measuring range and a background for negative sera below the conventional cut-off value. Serum testing was performed according to the recommendations of the immunoassay system manufacturer (Pharmacia Diagnostics AB, Uppsala, Sweden). Statistical parameters attesting the quality of the assays were calculated using system software.

### Immunoblotting

Twenty weed pollen allergic patients from Austria, thirty-two from Italy and (for control purposes) one nonatopic individual, were diluted 1:10 in buffer A ( 50 mM Na phoshate, pH 7.5, 0.5 % w/v bovine serum albumin (BSA), 0.5 % v/v Tween 20, 0.05 % NaN₃).

Sera were exposed to nitrocellulose-blotted mugwort and *Parietaria* pollen extracts. Bound IgE was detected with ¹²⁵J-labeled anti-human IgE antibodies (Valenta et al. 1992) and visualized by autoradiography using KODAK X-OMAT films and intensifying screens (Kodak, Heidelberg, Germany).

### IgE inhibition experiments

The presence of cross-reactive allergens/epitopes in mugwort and *Parietaria* extracts were investigated by IgE immunoblot inhibition experiments. Six mugwort allergic patients from the Austrian population were diluted 1/10 and preadsorbed with 100 µg/ml mugwort extract, 100 µg/ml *Parietaria* extract, 10 µg/ml rPhl p 7, 10 µg/ml
Bet v 2 and for control purposes with 10 µg/ml BSA overnight at 4° C. Preadsorbed sera were then exposed to nitrocellulose-blotted mugwort and *Parietaria* extract. Bound IgE Abs were detected with ¹²⁵J-labeled anti-human IgE antibodies (Pharmacia, Uppsala, Sweden).

### Skin prick testing

After informed consent was obtained, skin-prick tests were performed on the forearms of ten weed pollen allergic patients, and for control purposes of a nonallergic individual.
Drops of ragweed pollen extract, mugwort pollen extract, *Parietaria* pollen extract, histamin (1 µg/ml), and sodium chloride solutions (Soluprick; ALK, Horsholm, Sweden) were pricked with sterile lancets (ALK) as described (Vrtala et al. 1997). The skin reactions were recorded 20 min after testing by photography and by transferring the ball point pen-surrounded wheal area with scotch tape to paper. The mean wheal diameters displayed in Table 5 were determined as follows: Dm = (D1 + D2)/2. D1 and D2 represent the maximal longitudinal and transversal diameter of the wheal reaction in mm, respectively.

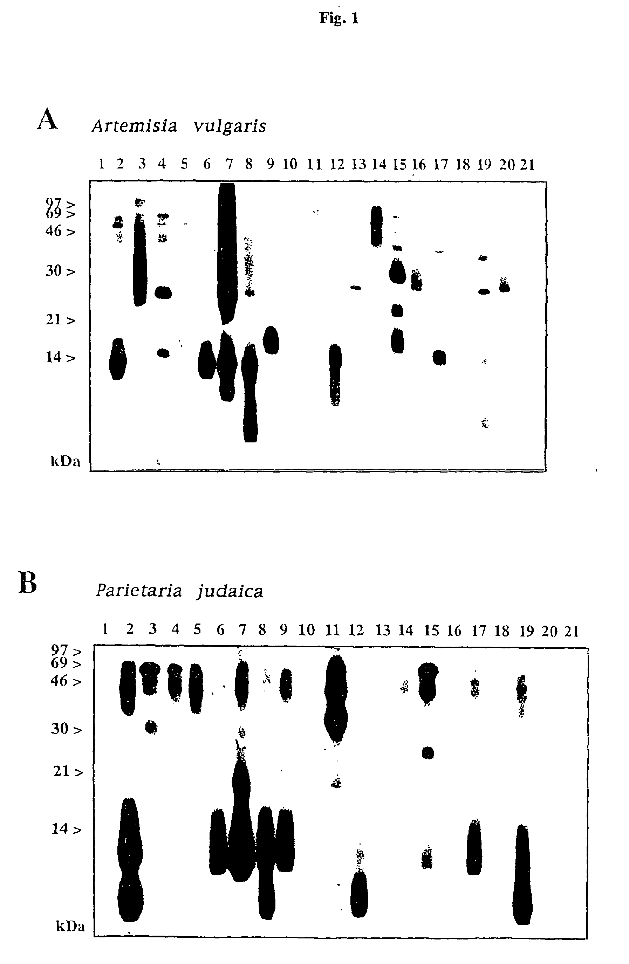

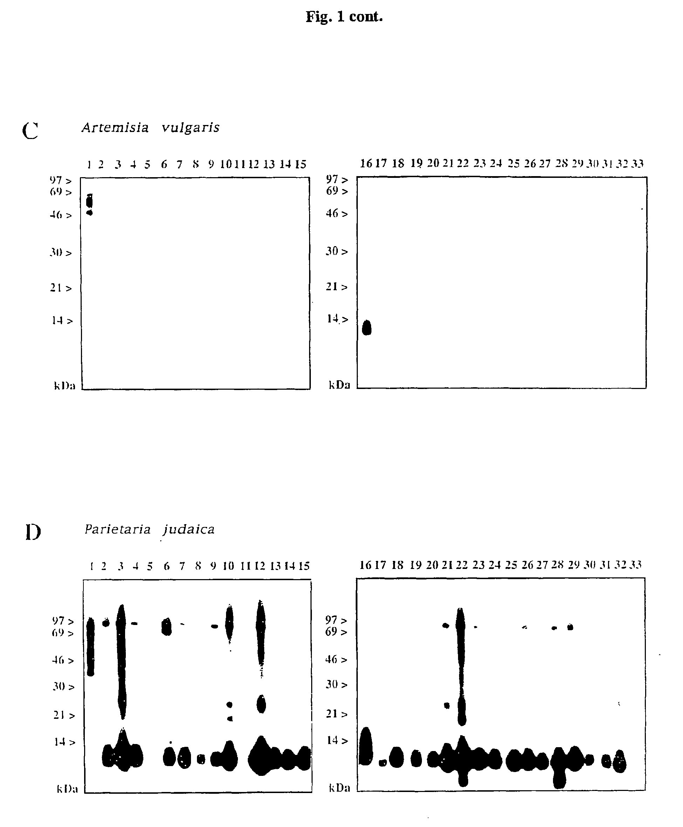

**Fig. 1****:** Different IgE reactivity profiles of Austrian and Mediterranean sera to blotted *Parietaria* extract.
Serum IgE reactivity of weed pollen allergic patients from Austria (A, B; n = 21) and Italy (C, D; n = 33) was tested to nitrocellulose-blotted mugwort (A, C) and parietaria (B, D) pollen extracts. Sixteen out of 20 Austrian weed pollen allergic patients showed IgE reactivity to proteins of different molecular weights to blotted *Parietaria* pollen extract (B).

In contrast, the mediterranean population shows a different reactivity-profile. Most patients reacted to Par j 2 at 10 kDa.

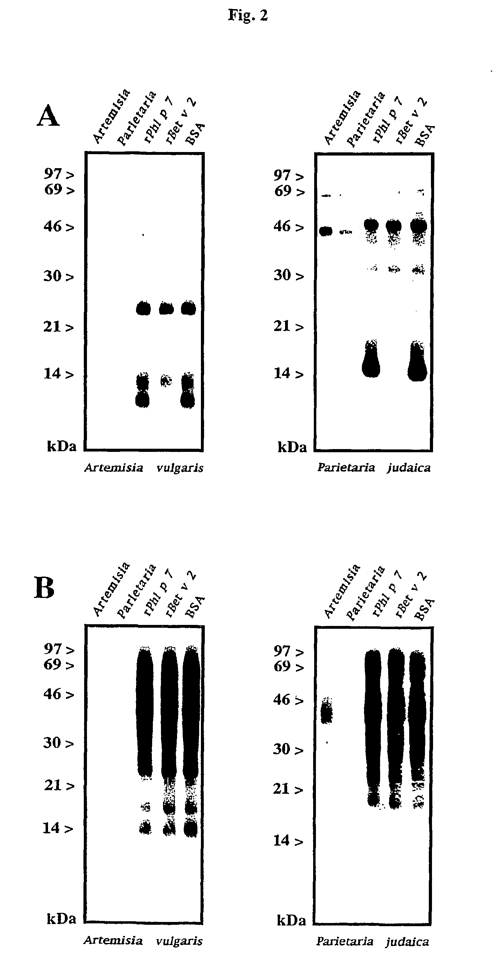

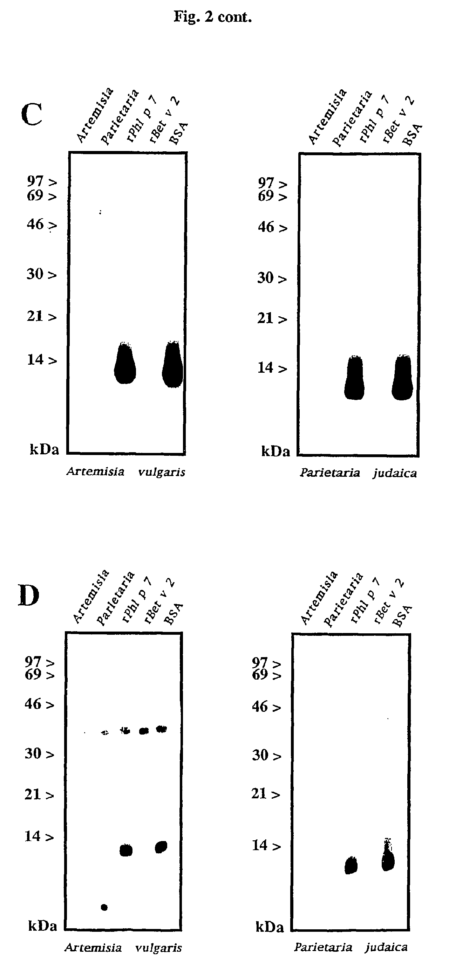

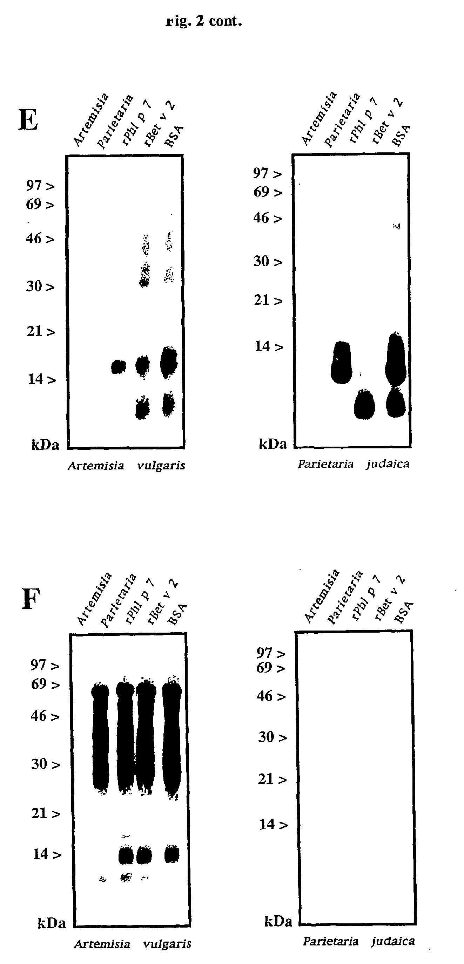

**Fig 2****.:** IgE inhibition experiment.
Six sera (A, B, C, D, E, F) from Austrian weed pollen allergic patients were preincubated either with mugwort pollen extract, *Parietaria* pollen extract, rPhl p 7, rBet v 2 or BSA and then exposed to nitrocellulose-blotted mugwort and *Parietaria* pollen extract. Mugwort and parietaria pollen extracts contain cross-reactive allergens of high molecular weight (25-100 kDa), profilin and two EF-hand allergens.

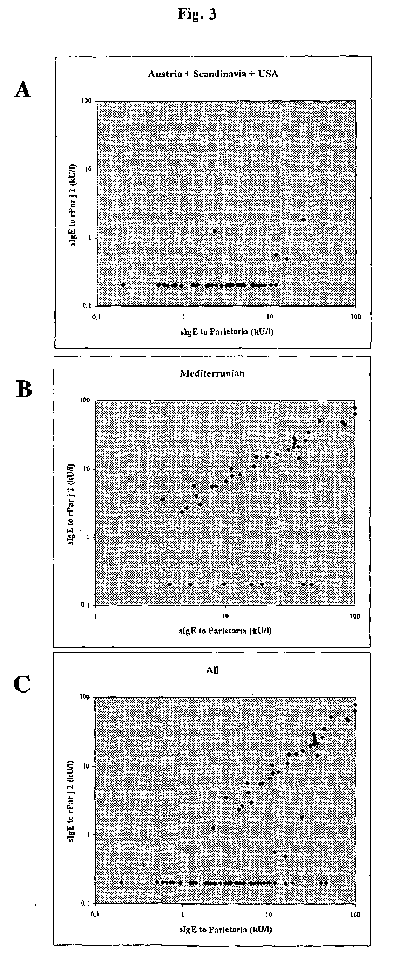

**Fig. 3****:** Quantitative analysis of serum IgE antibody binding to crude extract of *Parietaria* pollen and the allergen component rPar j 2.
Graphs are based on IgE antibody concentrations detailed in Tables 1-3.
Serum IgE to Par j 2 was measured for three groups of individuals, A = Austrian, Scandinavian and American, B = Mediterranean, and C = All these together.
The results show predominant sensitation to rPar j 2 in the patient population that originates from the region where *Parietaria* occurs, and virtually no sensitation to this protein in the populations that live outside the natural distribution area of Parietaria.

**Tables 1-4.** Detection of IgE antibodies to ragweed, mugwort, *Parietaria* and rPar j 2 by CAP RAST measurements in sera from different weed pollen allergic populations.

CAP RAST measurements show that almost all weed pollen allergic patients from Scandinavia, USA and Austria contain IgE antibodies to *Parietaria* pollen extract. No serum from Scandinavia and USA and only four (9.5 %) Austrian sera, reacted with rPar j 2. By contrast, 81 % of the mediterranean sera contained IgE anti-rPar j 2.

**Table 1**

| **Austria** | | | | |
|---|---|---|---|---|
| **specific IgE:kUA/I** | | | | |
| **patient** | **ragweed** | **mugwort** | **Parietaria** | **rPar j 2** |
| 1 | neg | 2.41 | neg | neg |
| 2 | 10 | 4.18 | 5 | neg |
| 3 | 0.9 | 5.34 | 0.80 | neg |
| 4 | 4.72 | 3.26 | 0.66 | neg |
| 5 | 0.68 | 2.7 | 0.52 | neg |
| 6 | 17.8 | 4.86 | 0.78 | neg |
| 7 | 53.4 | 46.7 | 6.99 | neg |
| 8 | 20.2 | 14.9 | 8.73 | neg |
| 9 | 1.15 | 2.23 | 0.60 | neg |
| 10 | 0.97 | 6.45 | neg | neg |
| 11 | 1.06 | 2.4 | 2.28 | 1.25 |
| 12 | 14.9 | 7.68 | 4.35 | neg |
| 13 | 2.46 | neg | neg | neg |
| 14 | 3.62 | 2.6 | 0.92 | neg |
| 15 | 23 | 7.92 | 1.33 | neg |
| 16 | 1.99 | 12.3 | neg | neg |
| 17 | 4.83 | 2.27 | 0.74 | neg |
| 18 | neg | 2.03 | neg | neg |
| 19 | 5.49 | 3.24 | 3.13 | neg |
| 20 | 0.83 | 2.21 | neg | neg |
| 21 | 2.27 | 2.32 | 0.94 | neg |
| 22 | 4.34 | 4.75 | 2.37 | neg |
| 23 | 5.29 | 5.71 | 11.9 | neg |
| 24 | 3.52 | 12.8 | neg | neg |
| 25 | 2.17 | 0.8 | 1.26 | neg |
| 26 | 3.92 | 3.56 | 1.84 | neg |
| 27 | 3.23 | 2.39 | 4.74 | neg |
| 28 | 21.6 | 19.4 | 15.9 | 0.49 |
| 29 | 2.95 | 10.6 | neg | neg |
| 30 | 5.1 | 3.75 | 1.84 | neg |
| 31 | 4.14 | 3.06 | 1.85 | neg |
| 32 | neg | 3.02 | neg | neg |
| 33 | 5.24 | 3.33 | 3.70 | neg |
| 34 | > 100 | 15.2 | 11.8 | 0.56 |
| 35 | 17.3 | 37.3 | neg | neg |
| 36 | 7.14 | 4.03 | 4.81 | neg |
| 37 | 1.19 | 18.5 | neg | neg |
| 38 | 36 | 9.21 | 25 | 1.80 |
| 39 | 6.23 | 6.51 | 1.44 | neg |
| 40 | 0.39 | 5.95 | neg | neg |
| 41 | 6.94 | 5.3 | 2.14 | neg |
| 42 | 11.2 | 6.17 | 4.22 | neg |
| **average** | **10.72** | **7.79** | **4.42** | **1.03** |

**Table 2**

| **Scandinavia** | | | | |
|---|---|---|---|---|
| **specific IgE:kUA/l** | | | | |
| **patient** | **ragweed** | **mugwort** | **parietaire** | **rPar j 2** |
| 1 | 14.82 | 21.22 | 2 | neg |
| 2 | 6.66 | 24.74 | 1.94 | neg |
| 3 | 2.75 | 4.81 | 6.48 | neg |
| 4 | 3.30 | 2.29 | 2.77 | neg |
| 5 | 10.95 | 5.54 | 7.50 | neg |
| 6 | 5.15 | 3.63 | 3.53 | neg |
| 7 | 9.70 | 6.43 | 8.76 | neg |
| 8 | 5.48 | 3.70 | 2.36 | neg |
| **average** | **7.35** | **9.05** | **4.42** | **-** |
| | | | | |

| **USA** | | | | |
|---|---|---|---|---|
| **specific IgE:kUA/l** | | | | |
| **patient** | **ragweed** | **mugwort** | **parietaire** | **rPar j 2** |
| 1 | neg | 0.45 | 2.35 | neg |
| 2 | 13.49 | 7.47 | 4.21 | neg |
| 3 | 3.06 | 1.72 | 1.93 | neg |
| 4 | 0.39 | neg | 3.47 | neg |
| 5 | 9.09 | 3.89 | 7.62 | neg |
| 6 | 3.03 | 1.86 | 5.12 | neg |
| 7 | 1.95 | 1.37 | 3.22 | neg |
| 8 | 111.11 | 5.10 | 4.31 | neg |
| 9 | 8.37 | 12.72 | 3.39 | neg |
| 10 | 22.16 | 11.01 | 3.19 | neg |
| 11 | 8.03 | 3.91 | 4.62 | neg |
| 12 | 3.45 | 4.35 | 3.14 | neg |
| 13 | 8.39 | 6.63 | 8.05 | neg |
| 14 | 14.16 | 5.99 | 10.29 | neg |
| 15 | 21.52 | 5.64 | 6.37 | neg |
| 16 | 42.60 | 9.71 | 4.97 | neg |
| 17 | 5.00 | 4.84 | 3.37 | neg |
| 18 | 90.17 | 6.80 | 6.92 | neg |
| **average** | **21.53** | **5.5** | **4.81** | - |

**Table 3**

| **Mediterranean** | | | | |
|---|---|---|---|---|
| **specific IgE:kUA/l** | | | | |
| **patient** | **ragweed** | **mugwort** | **parietaire** | **rPar j 2** |
| 1 | 4.44 | 3.01 | 3.73 | neg |
| 2 | neg | neg | 16.72 | 10.97 |
| 3 | 1.55 | neg | 113.79 | 64.19 |
| 4 | neg | 0.53 | 80.52 | 48.67 |
| 5 | 0.67 | neg | 4.60 | 2.34 |
| 6 | 2.92 | 0.37 | 37.33 | 14.48 |
| 7 | neg | neg | 17.36 | 14.89 |
| 8 | neg | neg | 3.29 | 3.53 |
| 9 | 0.74 | 1.07 | 5.65 | 5.71 |
| 10 | neg | neg | 53.65 | 51.61 |
| 11 | neg | 0.41 | 19.22 | neg |
| 12 | neg | neg | 84.12 | 45.52 |
| 13 | neg | 0.51 | 6.37 | 2.97 |
| 14 | neg | neg | 25.22 | 16.27 |
| 15 | neg | neg | 12.93 | 8.28 |
| 16 | 5.61 | 2.89 | 5.35 | neg |
| 17 | neg | neg | 21.10 | 15.04 |
| 18 | neg | neg | 11.08 | 10.26 |
| 19 | neg | neg | 8.34 | 5.60 |
| 20 | neg | neg | 11.33 | 7.84 |
| 21 | neg | neg | 33.89 | 20.92 |
| 22 | 1.92 | neg | 42.24 | 25.89 |
| 23 | 2.44 | 1.21 | 34.20 | 28.76 |
| 24 | neg | neg | 10.14 | 6.63 |
| 25 | neg | neg | 36.77 | 21.40 |
| 26 | 1.91 | neg | 30.90 | 19.46 |
| 27 | neg | neg | 7.90 | 5.53 |
| 28 | 0.95 | neg | 34.41 | 23.71 |
| 29 | neg | neg | 44.36 | 34.96 |
| 30 | neg | neg | 5.92 | 4.07 |
| 31 | neg | neg | 5.00 | 2.66 |
| 32 | neg | neg | 34.93 | 26.08 |
| 33 | 2.11 | neg | 122.59 | 78.49 |
| 34 | 33.42 | 11.67 | 46.94 | neg |
| 35 | 22.38 | 8.19 | 15.92 | neg |
| 36 | 45.24 | 23.90 | 40.74 | neg |
| 37 | 14.17 | 11.67 | 9.74 | neg |
| **average** | **9.36** | **5.45** | **29.95** | **20.89** |

**Table 4**

| | | **% of parietaire** | **% of rPar j 2** | | **average** | **IgE for** | |
|---|---|---|---|---|---|---|---|
| **origin** | **n** | **positive sera** | **reactive sera** | **ragweed** | **mugwort** | **parietaire** | **rPar j 2** |
| Austria | 42 | 71 | 9.5 | 10.72 | 7.79 | 4.42 | 1.03 |
| Scandinavia | 8 | 100 | - | 7.35 | 9.05 | 4.42 | - |
| USA | 18 | 100 | - | 21.53 | 5.5 | 4.81 | - |
| Mediterranian | 37 | 100 | 81 | 9.36 | 5.45 | 29.95 | 20.89 |

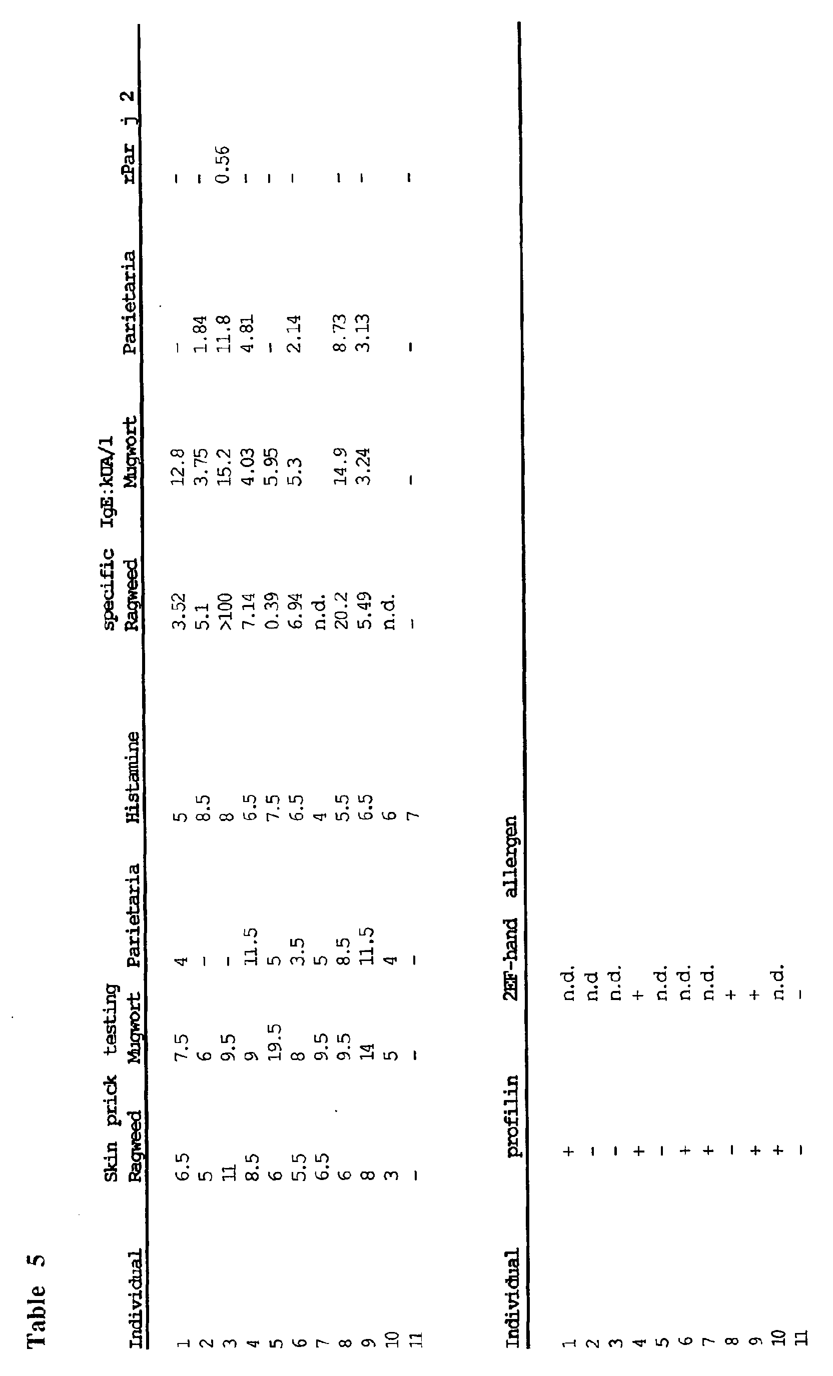

### REFERENCES

1. Duro, G., Colombo, P., Costa, M. A., Izzo, V., Porcasi, R., Di Fiore, R., Locorotondo, G., Mirisola, M. G., Cocchiara, R. and Gerachi, D. (1996). cDNA cloning, sequence analysis and serological characterization of Par j 2.0101, a new major allergen of the Parietaria judaica pollen. FEBS Lett. 399:295-298.
2. Costa, M. A., Duro, G., Izzo, V., Colombo, P., Mirisola, M. G., Locorotondo, G., Cocchiara, R. and Gerachi, D. (2000). The IgE-binding epitopes of rPar j 2, a major allergen of Parietaria judaica pollen, are heterogeneously recognized among allergic subjects. Allergy 55:246-250.
3. Bradford, M.M., A rapid and sensitive method for the quantification of microgram quantities of protein-dye-binding. Anal. Biochem. 1976, 72: 248-254.
4. Fling, S. P. and Gregerson, D.S., Peptide and protein molecular weight determination by electrophoresis using a high-molarity Tris buffer system without urea. Anal. Biochem. 1986, 155: 83-88.
5. Towbin, H., Staehelin, T. and Gordon, J., Electrophoretic transfer of proteins from polyacrylamide gels to nitrocellulose sheets: procedure and some applications. Proc. Natl. Acad. Sci. USA 1979. 76: 4350-4354.
6. Valenta, R; Duchene-M; Ebner-C; Valent-P; Sillaber-C; Devillor-P; Ferreira-F; Tejkl-M; Edelmann-H; Kraft-D; et al., Profilins constitute a novel family of functional plant pan allergens, J-Exp-Med. 1992 Feb. 1; 175(2): 377-85

## Claims

1. Use of a pure allergen component selected from the group consisting of Par j 1 and Par j 2 to serologically identify the actual sensitizing allergen source among a variety of possible allergen sources containing cross-reactive proteins or epitopes.

2. Use according to claim 1 for selection of treatment of a disorder involving an allergen.

3. Use according to any of claim 1 or 2, wherein the allergen component is synthetic, recombinant or native.

## Patentansprüche

1. Verwendung eines reinen Allergenbestandteils ausgewählt aus der Gruppe bestehend aus Par j 1 und Par j 2, um die eigentliche sensibilisierende Allergenquelle aus einer Auswahl von möglichen Allergenquellen, die kreuzreaktive Proteine oder Epitope enthalten, serologisch zu identifizieren.

2. Verwendung gemäß Anspruch 1, zur Auswahl einer Behandlung einer Erkrankung, die mit einem Allergen einhergeht.

3. Verwendung gemäß Anspruch 1 oder 2, wobei der Allergenbestandteil synthetisch, rekombinant oder natürlich ist.

## Revendications

1. Utilisation d'un composant allergène pur choisi dans le groupe constitué par Par j 1 et Par j 2 pour identifier sérologiquement la source d'allergène sensibilisant effective parmi une variété de sources d'allergène possibles contenant des protéines ou épitopes à réactivité croisée.

2. Utilisation selon la revendication 1, pour le choix d'un traitement d'un trouble faisant intervenir un allergène.

3. Utilisation selon l'une quelconque des revendications 1 ou 2, dans laquelle le composant allergène est synthétique, recombinant ou natif.
